# EUROPÄISCHE PATENTSCHRIFT

(11) **EP 2 865 346 B1**
(45) Veröffentlichungstag und Bekanntmachung des Hinweises auf die Patenterteilung: **19.07.2017**
(21) Anmeldenummer: 14189857.7
(22) Anmeldetag: 22.10.2014
(51) Int. Cl.: A61B 17/70

(54) **Wirbelsäulenstabilisierungssystem, medizinisches Instrumentarium und medizinische Vorrichtung zum parallelen Ausrichten medizinischer Instrumente**
Spinal stabilization system, medical equipment and medical device for the parallel alignment of medical instruments
Système de stabilisation de colonne vertébrale, instrument médical et dispositif médical pour l'alignement parallèle des instruments médicaux

(30) Priorität: 23.10.2013 DE 102013111683
(43) Veröffentlichungstag der Anmeldung: 29.04.2015
(73) Patentinhaber: AESCULAP AG, 78532 Tuttlingen (DE)
(72) Erfinder: Peukert, Andrea, 78532 Tuttlingen (DE); Hoefer, Fabian, 78532 Tuttlingen (DE)
(74) Vertreter: Hoeger, Stellrecht & Partner Patentanwälte mbB

(56) Entgegenhaltungen:
- US-A1- 2006 200 132
- US-A1- 2008 045 956
- US-A1- 2011 077 689
- US-A1- 2011 106 082

## Beschreibung

Die vorliegende Erfindung betrifft eine medizinische Vorrichtung zum parallelen Ausrichten von mindestens zwei eine Instrumentenlängsachse definierende medizinischen Instrumenten zum Halten und Handhaben eines chirurgischen Befestigungselements, welches Befestigungselement einen Befestigungsteil und einen relativ zum Befestigungsteil in einer Montagestellung beweglich gelagerten Halteteil für ein Verbindungselement umfasst, welche medizinischen Instrumente ein distales, mit dem Befestigungselement koppelbares Ende aufweisen, welche Vorrichtung mindestens zwei, jeweils eine Kopplungslängsachse definierende Kopplungseinrichtungen zum temporären Koppeln mit proximalen Enden der mindestens zwei medizinischen Instrumente umfasst, wobei die Vorrichtung einen Rahmen umfasst, wobei der Rahmen einen ersten und einen zweiten Rahmenabschnitt umfasst, welche parallel zueinander verlaufen, wobei die Kopplungslängsachsen der mindestens zwei Kopplungseinrichtungen parallel zueinander ausrichtbar sind, wobei die mindestens zwei Kopplungseinrichtungen in einer Montagestellung relativ zueinander und am Rahmen bewegbar gehalten sind und wobei sie in einer Ausrichtstellung relativ zueinander und am Rahmen unbewegbar gehalten sind.

Ferner betrifft die vorliegende Erfindung ein medizinisches Instrumentarium zum Implantieren eines Wirbelsäulenstabilisierungssystems, welches Instrumentarium mindestens zwei medizinische Instrumente zum Halten und Handhaben eines chirurgischen Befestigungselements umfasst, welches Befestigungselement einen Befestigungsteil und einen relativ zum Befestigungsteil in einer Montagestellung beweglich gelagerten Halteteil für ein Verbindungselement umfasst, welche medizinischen Instrumente ein proximales und ein distales, mit dem Befestigungselement koppelbares Ende aufweisen.

Und schließlich betrifft die vorliegende Erfindung auch ein Wirbelsäulenstabilisierungssystem umfassend mindestens zwei chirurgische Befestigungselemente und mindestens ein Verbindungselement, wobei mindestens eines der mindestens zwei chirurgischen Befestigungselemente einen Befestigungsteil, einen Halteteil mit einer Verbindungselementaufnahme und ein am Halteteil festlegbares Fixierelement zum Festlegen des Verbindungselements in der Verbindungselementaufnahme umfasst.

Ein Wirbelsäulenstabilisierungssystem und medizinische Instrumente der beschriebenen Art zum Halten und Handhaben chirurgischer Befestigungselemente sind beispielsweise aus der US 2011/0196426 A1 bekannt. Darin ist auch eine medizinische Vorrichtung zum parallelen Ausrichten von mindestens zwei eine Instrumentenlängsachse definierende medizinischen Instrumenten zum Halten und Handhaben eines chirurgischen Befestigungselements, welches Befestigungselement einen Befestigungsteil und einen relativ zum Befestigungsteil in einer Montagestellung beweglich gelagerten Halteteil für ein Verbindungselement umfasst, welche medizinischen Instrumente ein distales, mit dem Befestigungselement koppelbares Ende aufweisen, welche Vorrichtung mindestens zwei, jeweils eine Kopplungslängsachse definierende Kopplungseinrichtungen zum temporären Koppeln mit proximalen Enden der mindestens zwei medizinischen Instrumente umfasst, offenbart. Diese dient dazu, die mit chirurgischen Befestigungselementen in Form polyaxialer Pedikelschrauben gekoppelten Instrumente miteinander zu verbinden, um die Halteteile der Befestigungselemente in definierter Weise zueinander auszurichten. Die Instrumente werden insbesondere eingesetzt zum minimalinvasiven Festlegen des Wirbelsäulenstabilisierungssystems an einer menschlichen oder tierischen Wirbelsäule. Da bei minimalinvasivem Einsatz der Instrumente deren distale, mit den Halteteilen der Befestigungselemente gekoppelten Enden für einen Operateur nicht sichtbar sind, dieser aber Verbindungselemente, beispielsweise Verbindungsstäbe, in entsprechende Verbindungselementaufnahmen an den Halteteilen einführen muss, um diese daran festzulegen, so dass die Knochenschrauben in einer definierten Relativposition zueinander gehalten werden, ist es wünschenswert, die Ausrichtung der Verbindungselementaufnahmen zu kennen, auch wenn diese nicht direkt sichtbar sind. Um das Einführen des Verbindungselements zu erleichtern, dient die medizinische Vorrichtung. In der US 2011/0196426 A1 umfasst eine solche Vorrichtung einzelne Glieder, die direkt miteinander gekoppelt werden. Jedes Glied der Vorrichtung bildet eine Kopplungseinrichtung, die mit jeweils einem Instrument temporär koppelbar ist.

Nachteilig bei der bekannten medizinischen Vorrichtung ist insbesondere, dass diese nur umständlich, also insbesondere zeitintensiv, mit den medizinischen Instrumenten koppelbar ist. Ferner kann eine parallele Ausrichtung der Instrumentenlängsachsen der medizinischen Instrumente beim Einsatz der bekannten Vorrichtung nicht mit letzter Sicherheit garantiert werden.

Aus der US 2011/0106082 A1 sind Instrumente und Systeme zur Manipulation einer Wirbelsäule bekannt. Die US 2006/0200132 A1 offenbart Instrumente und Verfahren zur Manipulation eines Wirbels. In der US 2008/0045956 A1 sind minimalinvasive chirurgische Systeme beschrieben. Und aus der US 2011/0077689 A1 sind Verfahren und Vorrichtungen zum Manipulieren eines Wirbels bekannt.

Es ist daher eine Aufgabe der vorliegenden Erfindung, eine medizinische Vorrichtung, ein medizinisches Instrumentarium sowie ein Wirbelsäulenstabilisierungssystem der eingangs beschriebenen Art so zu verbessern, dass das Einführen eines Verbindungselements in Verbindungselementaufnahmen von Halteteilen mehrerer Befestigungseinrichtungen vereinfacht wird.

Diese Aufgabe wird bei einer medizinischen Vorrichtung der eingangs beschriebenen Art erfindungsgemäß dadurch gelöst, dass der Rahmen modular ausgebildet ist und mindestens eine Rahmenabschnittsverlängerung zum Verlängern der ersten und zweiten Rahmenabschnitte umfasst und dass die mindestens eine Rahmenabschnittsverlängerung mit dem ersten und/oder zweiten Rahmenabschnitt lösbar verbindbar ist.

Die erfindungsgemäß weitergebildete medizinische Vorrichtung ermöglicht es insbesondere, medizinische Instrumente der eingangs beschriebenen Art definiert und zuverlässig parallel auszurichten und zu halten. Insbesondere ist es möglich, zwei oder mehr Kopplungseinrichtungen am Rahmen anzuordnen, und zwar in Abhängigkeit von der Zahl der genutzten medizinischen Instrumente zum Halten und Handhaben der Befestigungselemente. Anders als bei der aus der US 2011/0196426 A1 bekannten medizinischen Vorrichtung lassen sich Abstände zwischen den Kopplungseinrichtungen insbesondere in der Montagestellung ändern und in der Ausrichtstellung optional so sichern, dass eine Bewegung der medizinischen Instrumente nicht oder nur erschwert möglich ist. Eine optimale parallele Ausrichtung der Instrumente mit der medizinischen Vorrichtung ermöglicht eine präzise Bestimmung einer Länge und Orientierung beziehungsweise Krümmung des Verbindungselements, das an den implantierten Befestigungselementen in deren Verbindungselementaufnahmen festgelegt werden soll. Auf einfache Weise lassen sich medizinische Instrumente parallel zueinander ausrichten, da der Rahmen einen ersten und einen zweiten Rahmenabschnitt umfasst, welche parallel zueinander verlaufen. Insbesondere können auch zwei Paare parallel zueinander verlaufender Rahmenabschnitte vorgesehen sein. Diese können insbesondere längs und quer zueinander verlaufende Abschnitte des Rahmens bilden. Gemäß der Erfindung ist vorgesehen, dass der Rahmen modular ausgebildet ist und mindestens eine Rahmenabschnittsverlängerung zum Verlängern der ersten und zweiten Rahmenabschnitte umfasst und dass die mindestens eine Rahmenabschnittsverlängerung mit dem ersten und/oder zweiten Rahmenabschnitt lösbar verbindbar ist. Mit einer oder auch zwei Rahmenabschnittsverlängerungen lässt sich der Rahmen vergrößern. Insbesondere können die Rahmenabschnittsverlängerungen ähnlich wie die Rahmenabschnitte ausgebildet sein, um daran Kopplungseinrichtungen oder deren Kopplungsglieder anzuordnen. Ein solcher modularer Rahmen ermöglicht es daher, je nach Bedarf den Rahmen zu vergrößern, um die für einen chirurgischen Eingriff benötigte Zahl medizinischer Instrumente parallel zueinander auszurichten. Der Rahmen kann also individuell an den jeweiligen Bedarf in seiner Größe angepasst werden, so dass er nur minimal den Zugang und die Sicht auf den Operationssitus für den Operateur beeinträchtigt.

Günstig ist es, wenn jede der Kopplungseinrichtungen mindestens zwei Kopplungsglieder umfasst, die in der Montagestellung relativ zueinander bewegbar und am Rahmen bewegbar gehalten sind und die in der Ausrichtstellung relativ zueinander unbewegbar und am Rahmen unbewegbar gehalten sind. Mit derart ausgebildeten Kopplungseinrichtungen können medizinische Instrumente in der Ausrichtstellung definiert und sicher gehalten werden. Ein Anlegen der medizinischen Vorrichtung an zwei oder mehr medizinische Instrumente ist besonders einfach, da die mindestens zwei Kopplungsglieder, insbesondere können es nur zwei sein, in der Montagestellung relativ zueinander bewegbar sind. Beispielsweise können sie voneinander weg bewegbar sein, um das Einführen eines medizinischen Instruments zwischen diese zu erleichtern. Insbesondere ist es so möglich, mit der medizinischen Vorrichtung alle medizinischen Instrumente in einem Schritt zu fassen und parallel zueinander auszurichten.

Um eine optimale Anpassung und Ausrichtung der Instrumente zu erreichen, ist es vorteilhaft, wenn jedes Kopplungsglied eine Instrumentenanlagefläche aufweist, die an eine äußere Kontur des medizinischen Instruments im Bereich von dessen proximalem Ende angepasst ist. So kann ein Verkippen beziehungsweise ein unerwünschtes Neigen des Instruments relativ zur Kopplungseinrichtung vermieden werden, was insbesondere zu einer nicht parallelen Ausrichtung der mindestens zwei an der medizinischen Vorrichtung gehaltenen Instrumente führen kann. Günstigerweise erstreckt sich die Instrumentenanlagefläche in einer Richtung parallel zur Instrumentenlängsachse auf einer Länge, die etwa einer halben Breite der Instrumentenanlagefläche quer zur Instrumentenlängsachse entspricht. Für eine besonders gute und genaue Ausrichtung der Instrumente ist es vorteilhaft, je größer die Erstreckung der Instrumentenanlagefläche parallel zur Instrumentenlängsachse bezogen auf die Breite der Instrumentenanlagefläche ist.

Insbesondere ist es vorteilhaft, wenn die Instrumentenanlagefläche in Form eines hohlzylindrischen Wandflächenabschnitts ausgebildet ist. Ein solcher hohlzylindrischer Wandflächenabschnitt, der vorzugsweise einen Innendurchmesser definiert, welcher einem Außendurchmesser des zu haltenden und auszurichtenden Instruments entspricht, kann insbesondere großflächig zum Anlegen an eine zylindrische Außenfläche des Instruments genutzt werden.

Günstig ist es, wenn der Rahmen mindestens zwei Rahmenquerabschnitte umfasst, welche parallel oder im Wesentlichen parallel zueinander verlaufen, wenn die ersten und zweiten Rahmenabschnitte Rahmenlängsabschnitte bilden und wenn die mindestens zwei Rahmenquerabschnitte die Rahmenlängsabschnitte, insbesondere freie Enden derselben, miteinander verbinden. Auf diese Weise kann insbesondere ein rechteckiger oder im Wesentlichen rechteckiger Rahmen ausgebildet werden. Vorzugsweise sind Abstände der Rahmenlängsabschnitte an Querschnitte der auszurichtenden Instrumente angepasst, so dass diese zwischen die Rahmenlängsabschnitte eingeführt werden können. Insbesondere können so alle medizinischen Instrumente innerhalb der von den Rahmenlängsabschnitten und Rahmenquerabschnitten begrenzten freien Fläche des Rahmens gehalten und ausgerichtet werden. Die Rahmenquerabschnitte können insbesondere den Rahmen quasi verschließen. Sie können optional ausgebildet sein zum Halten von Kopplungsgliedern. Allerdings können sie auch nur zum Verbinden der Rahmenlängsabschnitte und zum Stabilisieren des Rahmens dienen.

Vorteilhaft ist es, wenn die mindestens zwei Rahmenquerabschnitte Rahmenquerabschnittslängsachsen definieren, welche quer, insbesondere senkrecht, zu Rahmenlängsabschnittslängsachsen der mindestens zwei Rahmenlängsabschnitte verlaufen. Insbesondere bei einer rechtwinkligen Ausrichtung der Rahmenquerschnittslängsachsen und der Rahmenlängsabschnittslängsachsen lässt sich ein insgesamt rechteckiger oder im Wesentlichen rechteckiger Rahmen ausbilden.

Auf einfache Weise lässt sich der Rahmen modular ausbilden, wenn eine Rahmenabschnittsverbindungseinrichtung zum lösbaren Verbinden von den Rahmen ausbildenden Teilen desselben umfasst. Die Rahmenabschnittsverbindungseinrichtung ermöglicht es insbesondere, Rahmenlängsabschnitte mit Rahmenquerabschnitten und optional auch Rahmenabschnittsverlängerungen temporär miteinander zu verbinden, um so einfach und sicher einen Rahmen benötigter Größe auszubilden. Die Rahmenabschnittsverbindungseinrichtung kann insbesondere in Form einer Rast- oder Schnappverbindung ausgebildet sein, die zusammenwirkende Rast- oder Schnappglieder an den miteinander zu verbindenden Teilen des Rahmens aufweist.

Günstig ist es, wenn die Rahmenabschnittsverbindungseinrichtung erste und zweite zusammenwirkende Rahmenabschnittsverbindungsglieder umfasst, die in einer Rahmenverbindungsstellung kraft- und/oder formschlüssig miteinander in Eingriff stehen. Insbesondere können erste und zweite Rahmenabschnittsverbindungsglieder an den miteinander zu verbindenden Abschnitten des Rahmens angeordnet oder ausgebildet sein. Die Rahmenabschnittsverbindungsglieder können insbesondere in Form von Rast- oder Schnappgliedern ausgebildet sein, die kraft- und/oder formschlüssige Verbindungen ermöglichen.

Auf besonders einfache Weise lassen sich Teile des Rahmens miteinander koppeln, wenn die ersten und zweiten zusammenwirkenden Rahmenabschnittsverbindungsglieder in Form von Rahmenabschnittsverbindungsvorsprüngen und zu diesen korrespondierenden Rahmenabschnittsverbindungsaufnahmen ausgebildet sind. Auf einfache Weise lassen sich diese zusammenführen, wenn die Rahmenabschnittsverbindungsglieder Längsachsen definieren, die beispielsweise parallel zu Rahmenlängsabschnittslängsachsen oder Rahmenquerabschnittslängsachsen verlaufen.

Vorzugsweise sind die ersten und zweiten zusammenwirkenden Rahmenabschnittsverbindungsglieder an einem Rahmenabschnitt, an einem Rahmenlängsabschnitt, an einem Rahmenquerabschnitt und/oder an einer Rahmenabschnittsverlängerung angeordnet oder ausgebildet. Idealerweise sind die Rahmenabschnittsverbindungsglieder derart angeordnet, dass die genannten Teile im Wesentlichen beliebig miteinander verbindbar sind. Vorzugsweise sind die Rahmenabschnittsverbindungsglieder jeweils unlösbar mit einem der genannten Teile verbunden, so dass die Zahl freier Teile minimiert werden kann, insbesondere von kleinen Teilen. So kann eine Gefahr, dass Teile des Rahmens im Bereich des Operationssitus verloren gehen können, minimiert werden.

Günstigerweise sind den Rahmen ausbildende Teile, insbesondere die ersten und zweiten Rahmenabschnitte, einstückig ausgebildet. Auf diese Weise lässt sich die Zahl der den Rahmen ausbildenden Teile minimieren. Insbesondere ist es denkbar, den Rahmen aus lediglich zwei Teilen auszubilden, beispielsweise einer Einheit aus zwei Rahmenlängsabschnitten, die über einen Rahmenquerabschnitt dauerhaft verbunden sind, oder beispielsweise durch vier Teile, zwei Rahmenlängsabschnitte und zwei mit diesen lösbar verbindbare Rahmenquerabschnitte. Zum Vergrößern des Rahmens können dann beispielsweise zusätzlich zwei Rahmenabschnittsverlängerungen die beiden Rahmenlängsabschnitte verlängern. Die Rahmenabschnittsverlängerungen können auch als einstückig ausgebildete Einheit geformt sein, beispielsweise über einen Quersteg verbunden, so dass auch hier das Zusammenfügen der Teile und Erweitern des Rahmens bei Bedarf auf einfache Weise möglich ist.

Vorteilhafterweise umfasst die Vorrichtung eine Verbindungseinrichtung zum beweglichen Verbinden der Kopplungsglieder mit dem Rahmen. Eine solche Verbindungseinrichtung ermöglicht es, die Kopplungsglieder und damit die Kopplungseinrichtungen praktisch beliebig am Rahmen zu positionieren, um so den Rahmen und die Position der an diesen angeordneten Kopplungseinrichtungen in Abhängigkeit der jeweiligen operativen Situation an die medizinischen Instrumente, die ausgerichtet werden sollen, anzupassen.

Auf einfache Weise lässt sich die Verbindungseinrichtung ausbilden, wenn sie mindestens ein erstes Verbindungselement und mindestens ein zweites, mit dem mindestens einen ersten Verbindungselement zusammenwirkendes Verbindungselement umfasst, wenn der Rahmen das mindestens eine erste Verbindungselement umfasst und wenn das mindestens eine zweite Verbindungselement am Kopplungsglied angeordnet oder ausgebildet ist. Eine solche Ausgestaltung ermöglicht es insbesondere, das Kopplungsglied direkt am Rahmen oder einem der diesen ausbildenden Teile anzuordnen.

Günstig ist es, wenn das mindestens eine erste Verbindungselement in Form einer Verbindungsaufnahme ausgebildet ist und wenn das mindestens eine zweite Verbindungselement in Form eines Verbindungsvorsprungs ausgebildet ist. Derartige Verbindungselemente lassen sich auf einfache Weise kraft- und/ oder formschlüssig miteinander in Eingriff bringen, so dass die Kopplungsglieder am Rahmen einfach und definiert gehalten werden können. Insbesondere sind sie auf einfache Weise auch derart ausbildbar, dass eine Rotation derselben verhindert werden kann, beispielsweise durch entsprechende Wahl ihrer Form. Insbesondere könne sie von einer Rotationssymmetrie abweichende Querschnittsflächen und -formen aufweisen, so dass eine Rotation der Kopplungsglieder relativ zum Rahmen unmöglich ist.

Günstigerweise ist der Verbindungsvorsprung in der Montagestellung in oder an der Verbindungsaufnahme verschiebbar. So ist es insbesondere möglich, das Kopplungsglied relativ zum Rahmen in der Montagestellung auf einfache Weise zu verschieben, um es genau dort zu positionieren, wo es zum Aufnehmen eines Teils des auszurichtenden medizinischen Instruments benötigt wird.

Auf einfache Weise ausbilden lässt sich der Rahmen, wenn die Verbindungsaufnahme Teil einer Führungsschiene oder Führungsnut ist. So ist es insbesondere ausreichend, wenn lediglich eine Führungsschiene oder Führungsnut an einem Rahmenabschnitt ausgebildet oder angeordnet ist. Dies gestattet es, insbesondere zwei oder mehr Kopplungsglieder darin anzuordnen, in der Montagestellung zu verschieben und in der Ausrichtstellung zu fixieren.

Um zwei Kopplungsglieder zur Ausbildung einer Kopplungseinrichtung einfach, schnell und sicher parallel ausrichten zu können, ist es vorteilhaft, wenn die Verbindungseinrichtung zwei zueinander parallel verlaufende Verbindungsaufnahmen umfasst.

Um das Aufnehmen eines medizinischen Instruments in einer Kopplungseinrichtung zu vereinfachen, ist es vorteilhaft, wenn beiden eine Kopplungseinrichtung definierenden Kopplungsglieder relativ zueinander in einer Richtung quer zu einer von den mindestens einen ersten Verbindungselementen definierten Längsrichtung beim Übergang von der Ausrichtstellung in die Montagestellung voneinander weg und beim Übergang von der Montagestellung in die Ausrichtstellung aufeinander zu bewegbar sind. Mit anderen Worten kann so insbesondere ein Abstand zwischen den Kopplungsgliedern vergrößert werden beim Übergang von der Ausrichtstellung in die Montagestellung, so dass beispielsweise ein hülsenförmiger Instrumentenabschnitt des auszurichtenden medizinischen Instruments zwischen die Kopplungsglieder eingeführt werden kann.

Vorzugsweise umfasst die Kopplungseinrichtung eine Feststelleinrichtung zum Sichern einer Position und/oder Ausrichtung der Kopplungsglieder in der Ausrichtstellung. Mit der Feststelleinrichtung lässt sich so insbesondere verhindern, dass sich die Kopplungsglieder relativ zueinander in der Ausrichtstellung in unerwünschter Weise bewegen. Damit kann eine parallele Ausrichtung der mit der Vorrichtung gehaltenen Instrumente sichergestellt werden.

Günstigerweise umfasst die Feststelleinrichtung eine Klemm- oder Blockiereinrichtung zum klemmenden Festlegen der Kopplungsglieder in der Ausrichtstellung am Rahmen oder zum Blockieren einer Bewegung derselben relativ zueinander. Mit der Klemm- oder Blockiereinrichtung lassen sich somit die Kopplungsglieder insbesondere in der Ausrichtstellung relativ zueinander und/ oder relativ zum Rahmen festlegen.

Günstig ist es, wenn die Klemmeinrichtung mindestens ein Klemmglied umfasst zum klemmenden Festlegen der Kopplungsglieder in der Ausrichtstellung am Rahmen. Das mindestens eine Klemmglied kann insbesondere in Form eines elastischen Elements oder aber auch in Form eines inelastischen Körpers ausgebildet sein, welcher ein oder mehrere Kopplungsglieder klemmend am Rahmen oder einem Teil desselben blockiert. Insbesondere kann das Klemmglied auch eine Kraft ausüben, die davon abhängt, ob sich das Kopplungsglied in der Montagestellung oder in der Ausrichtstellung befindet.

Günstigerweise ist das mindestens eine Klemmglied in Form eines Federelements ausgebildet. Beispielsweise kann so das Kopplungsglied entgegen der Wirkung des Federelements von der Ausrichtstellung in die Montagestellung überführt werden, um das Kopplungsglied relativ zum Rahmen zu bewegen.

Auf besonders einfache Weise lässt sich die Klemmeinrichtung ausgestalten, wenn das Federelement in Form einer Blattfeder oder Schraubenfeder ausgebildet ist.

Gemäß einer weiteren bevorzugten Ausführungsform der Erfindung kann vorgesehen sein, dass mindestens ein Kopplungsglied jeder Kopplungseinrichtung entgegen der Wirkung des Klemmglieds von der Ausrichtstellung in die Montagestellung überführbar ist. Das Kopplungsglied, beispielsweise ein elastisches Element, insbesondere in Form eines Federelements, kann zum Beispiel derart angeordnet sein, dass es eine Kraft auf das Kopplungsglied so ausübt, dass zum Überführen des Kopplungsglieds von der Ausrichtstellung in die Montagestellung entgegen der Wirkung des Klemmglieds eine Kraft ausgeübt werden muss. So kann insbesondere vermieden werden, dass sich die Kopplungsglieder in unerwünschter Weise von selbst relativ zueinander oder relativ zum Rahmen bewegen.

Vorteilhaft ist es, wenn nur eines der beiden Kopplungsglieder einer Kopplungseinrichtung ein Klemmglied umfasst. Auf diese Weise kann der konstruktive Aufbau und auch der Herstellungs- und Montageaufwand der Vorrichtung minimiert werden. Selbstverständlich ist es auch möglich, beide Kopplungsglieder einer Kopplungseinrichtung mit einem Klemmglied auszustatten. Vorteilhafterweise ist das Klemmglied als Druck- oder Zugglied ausgebildet. Es kann also das Kopplungsglied in der Ausrichtstellung insbesondere durch Druck- oder Zugkräfte am Rahmen festliegen.

Vorteilhaft ist es, wenn mindestens eines der Kopplungsglieder einen Führungsabschnitt, einen Halteabschnitt und ein den Führungsabschnitt und den Haltabschnitt verbindendes Verbindungsglied aufweist. Insbesondere können beide Kopplungsglieder einer Kopplungseinrichtung derart ausgebildet sein. Durch diese Ausgestaltung lassen sich die jeweiligen Teile des Kopplungsglieds entsprechend ihrer Aufgabe optimieren, beispielsweise auch aus unterschiedlichen Materialien herstellen.

Besonders einfach und kompakt ausbilden lässt sich die Vorrichtung, wenn der Führungsabschnitt das zweite Verbindungselement ist oder umfasst. So ist es insbesondere möglich, dass der Führungsabschnitt nicht nur zum Führen des Kopplungsglieds am Rahmen genutzt werden kann, sondern insbesondere auch zum Verbinden desselben mit dem Rahmen.

Vorteilhaft ist es, wenn der Führungsabschnitt ausgebildet ist zum verdrehsicheren Verschieben am mindestens einen ersten Verbindungselement. Insbesondere kann die Ausbildung des Führungsabschnitts derart sein, dass eine Verdrehsicherung beziehungsweise ein verdrehsicheres Verschieben insbesondere in der Montagestellung möglich ist. Durch die verdrehsichere Ausbildung des Führungsabschnitts und des ersten Verbindungselements kann verhindert werden, dass eine von der Kopplungseinrichtung definierte Längsachse in unerwünschter Weise verkippt und so die an der Vorrichtung ausgerichteten Instrumente gegeneinander verkippt werden können.

Günstig ist es, wenn der Führungsabschnitt in der Verbindungsaufnahme parallel zur Längsrichtung verschiebbar gehalten ist. So kann das Kopplungsglied bei Bedarf in gewünschter Weise relativ zum Rahmen einfach und sicher verschoben werden, insbesondere in der Montagestellung.

Auf einfache Weise lässt sich ein Kopplungsglied in der Ausrichtstellung am Rahmen festlegen, wenn sich das Klemmglied einerseits am Halteabschnitt und andererseits an der Verbindungsaufnahme oder einerseits an der Verbindungsaufnahme und andererseits am Führungsabschnitt abstützt. Wo sich das Klemmglied vorzugsweise abstützt, hängt insbesondere von der Art des Klemmglieds ab, also ob dieses als Druckglied oder Zugglied ausgebildet ist.

Ferner kann es vorteilhaft sein, wenn die Blockiereinrichtung ein Blockierelement umfasst, welches in der Montagestellung in die Verbindungsausnehmung einschiebbar ist und in der Ausrichtstellung zwischen einer vom Halteabschnitt weg weisenden Seitenfläche des Führungsabschnitts und einer inneren Führungsfläche der Verbindungsausnehmung angeordnet ist. Beispielsweise kann das Blockierelement in Form eines inelastischen oder im Wesentlichen inelastischen Elements ausgebildet sein, beispielsweise in Form eines Metall- oder Kunststoffstreifens, welcher in einen zwischen dem Führungsabschnitt und der Verbindungsausnehmung ausgebildeten Spalt einschiebbar ist, um so eine Bewegung des Kopplungsglieds quer zur Längsrichtung zu unterbinden.

Vorteilhaft ist es, wenn das Blockierelement ausgebildet ist zum Blockieren einer Bewegung von mindestens zwei Kopplungsgliedern gleichzeitig. Insbesondere kann so auf einfache Weise die gesamte Vorrichtung in der Ausrichtstellung gesichert werden. Insbesondere ist es denkbar ein einziges Blockierelement zu nutzen, welches in eine Führungsnut oder Führungsschiene eingeschoben wird, um eine Bewegung der Kopplungsglieder quer zur Längsrichtung zu verhindern. Auf diese Weise kann vollständig auf Federelemente oder elastische Elemente verzichtet werden.

Die eingangs gestellte Aufgabe wird bei einem medizinischen Instrumentarium erfindungsgemäß dadurch gelöst, dass es eine der oben beschriebenen Vorrichtungen umfasst.

Das medizinische Instrumentarium weist dann ebenfalls die oben im Zusammenhang mit bevorzugten Ausführungsformen medizinischer Vorrichtung beschriebenen Vorteile auf. Es ermöglicht die einfache, sichere und präzise Implantation eines Wirbelsäulenstabilisierungssystems.

Die eingangs gestellte Aufgabe wird ferner bei einem Wirbelsäulenstabilisierungssystem der eingangs beschriebenen Art erfindungsgemäß dadurch gelöst, dass es eine der oben beschriebenen Vorrichtungen oder das oben beschriebene medizinische Instrumentarium umfasst.

Ein solches Wirbelsäulenstabilisierungssystem ermöglicht auf einfache und sichere Weise die Ausrichtung von Wirbeln einer Wirbelsäule relativ zueinander.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische Gesamtansicht eines ersten Ausführungsbeispiels einer medizinischen Vorrichtung mit drei an dieser parallel zueinander ausgerichteten medizinischen Instrumenten;
- Figur 2:: eine Ansicht der Anordnung aus Figur 1 in Richtung des Pfeils A;
- Figur 3:: eine schematische perspektivische Ansicht der medizinischen Vorrichtung aus Figur 2 beim Zusammenfügen von Rahmenlängsabschnitten mit einem Rahmenquerabschnitt;
- Figur 4:: eine Ansicht der Anordnung aus Figur 3 in Richtung des Pfeils B;
- Figur 5:: eine Schnittansicht längs Linie 5-5 in Figur 3;
- Figur 6:: eine schematische perspektivische Darstellung der um zwei Rahmenabschnittsverlängerungen verlängerten Vorrichtung aus Figur 3;
- Figur 7:: eine schematische perspektivische Explosionsdarstellung der Anordnung aus Figur 6;
- Figur 8:: eine Schnittansicht längs Linie 8-8 in Figur 6;
- Figur 9:: eine schematische perspektivische Anordnung eines zweiten Ausführungsbeispiels einer medizinischen Vorrichtung;
- Figur 10:: eine Ansicht der Anordnung aus Figur 9 in Richtung des Pfeils C;
- Figur 11:: eine Schnittansicht längs Linie 11-11 in Figur 9;
- Figur 12:: eine schematische perspektivische Darstellung eines dritten Ausführungsbeispiels einer medizinischen Vorrichtung;
- Figur 13:: eine Ansicht der Anordnung aus Figur 12 in Richtung des Pfeils D;
- Figur 14:: eine Schnittansicht längs Linie 14-14 in Figur 12; und
- Figur 15:: eine schematische, vergrößerte perspektivische Ansicht eines Kopplungsglieds der Anordnung aus Figur 12 mit einem Klemmglied in Form einer Blattfeder.

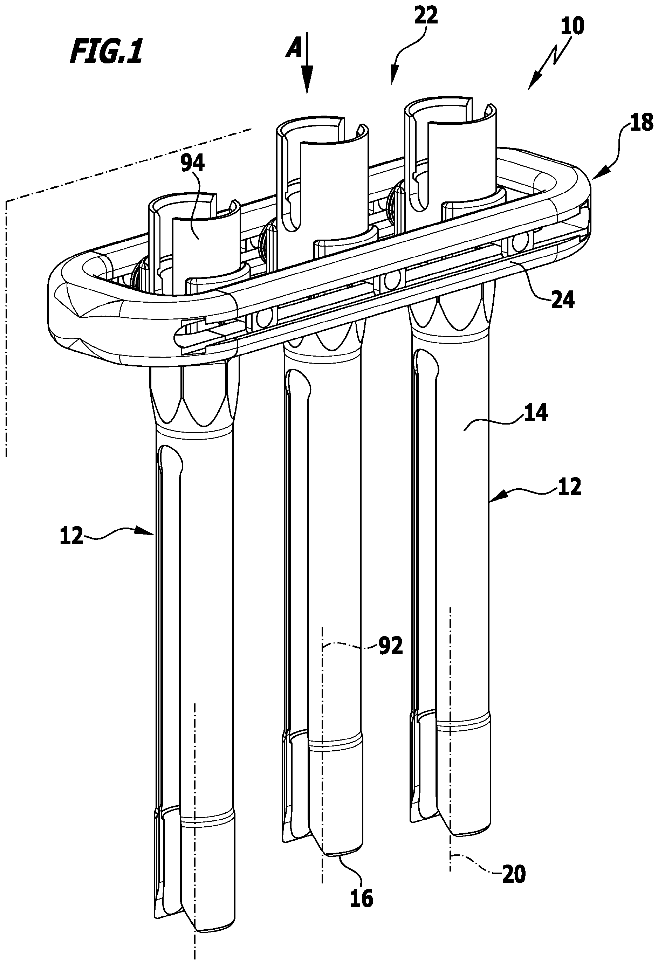

In Figur 1 ist beispielhaft ein Teil eines Wirbelsäulenstabilisierungssystems 10 umfassend mindestens zwei chirurgische Befestigungselemente und mindestens ein Verbindungselement schematisch dargestellt. In Figur 1 ferner abgebildet sind drei medizinische Instrumente 12 beziehungsweise ein Teil derselben in Form einer Außenhülse 14, welche mit dem in Figur 1 nicht dargestellten Befestigungselement, das beispielsweise in Form einer Knochenschraube, insbesondere einer polyaxialen Pedikelschraube, koppelbar ist, und zwar mit ihren distalen Enden 16. Das Wirbelsäulenstabilisierungssystem 10 umfasst ferner eine medizinische Vorrichtung 18 zum Ausrichten von Instrumentenlängsachsen 20 der Instrumente 12. Die Vorrichtung 18 bildet ferner einen Teil eines medizinischen Instrumentariums 22 zum Implantieren des Wirbelsäulenstabilisierungssystems 10, wobei das Instrumentarium 22 insbesondere die Instrumente 12 und die Vorrichtung 18 umfasst.

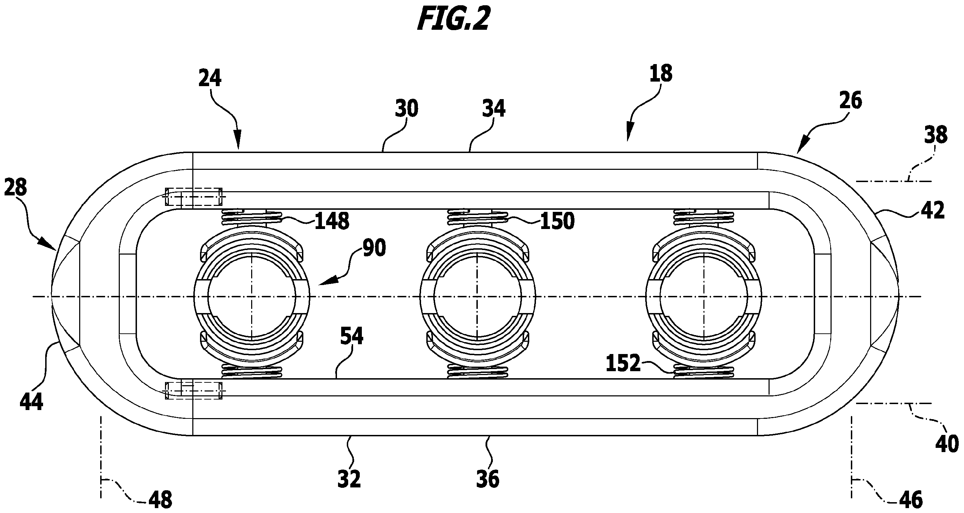

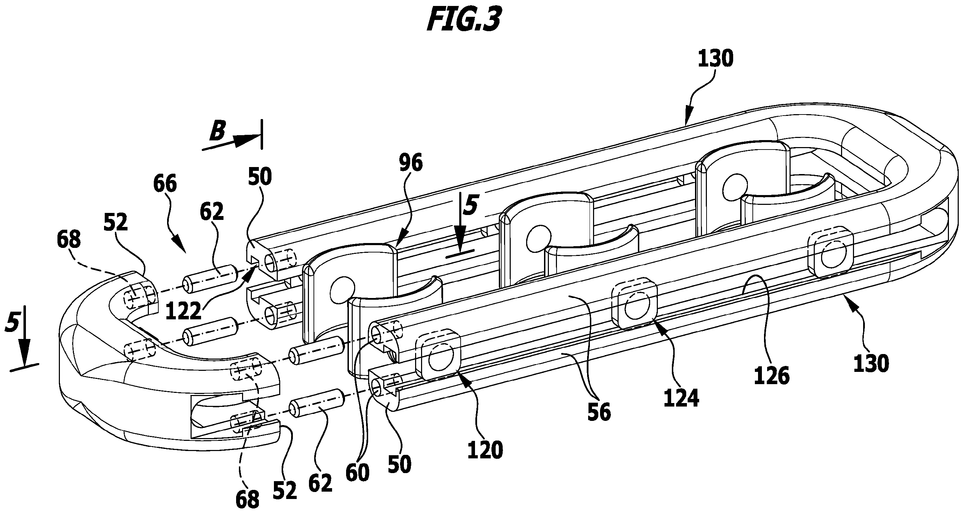

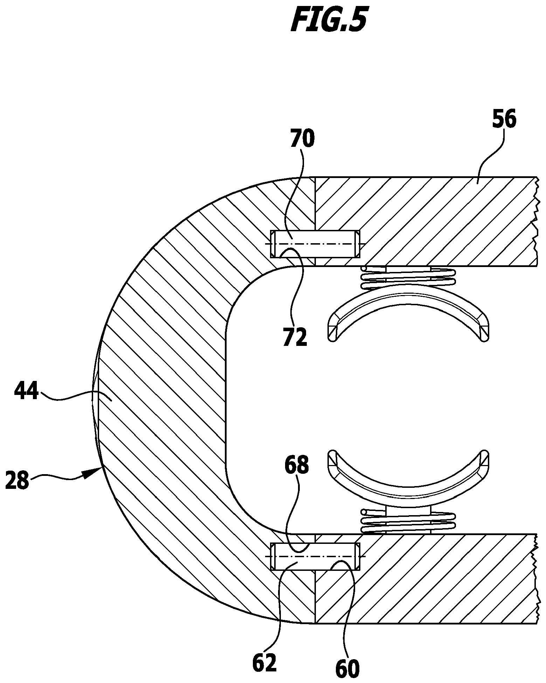

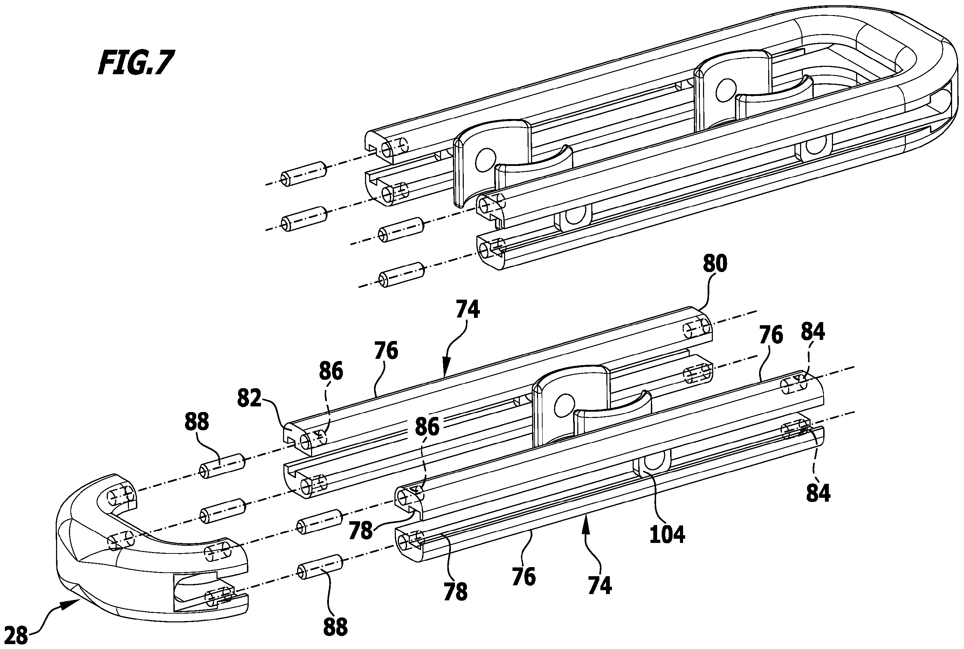

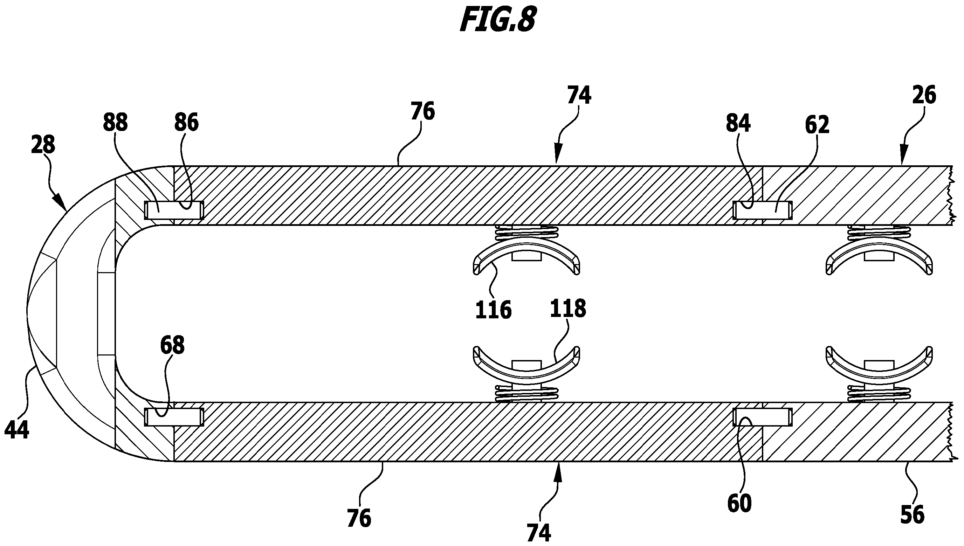

Der Aufbau der Vorrichtung 18 wird nachfolgend in Verbindung mit den Figuren 2 bis 8 im Einzelnen erläutert.

Die Vorrichtung 18 umfasst einen insgesamt mit dem Bezugszeichen 24 bezeichneten Rahmen, welcher aus mehreren Teilen gebildet ist. Das in den Figuren 2 bis 5 schematisch dargestellte Ausführungsbeispiel umfasst zwei Rahmenteile 26 und 28. Der erste Rahmenteil 26 umfasst zwei parallel zueinander verlaufende Rahmenabschnitte 30 und 32, die Rahmenlängsabschnitte 34 und 36 bilden, die parallel zueinander verlaufende Rahmenlängsabschnittslängsachsen 38 und 40 definieren. Die Rahmenlängsabschnitte 34 und 36 sind an ihrem einen Ende über einen Rahmenquerabschnitt 42 verbunden. Ein weiterer Rahmenquerabschnitt 44 ist spiegelsymmetrisch zum Rahmenquerabschnitt 42 angeordnet und verbindet die anderen freien Enden der Rahmenlängsabschnitte 34 und 36, so dass insgesamt ein geschlossener, im Wesentlichen rechteckiger Rahmen 24 gebildet wird.

Die Rahmenquerabschnitte 42 und 44 definieren jeweils Rahmenquerabschnittslängsachsen 46 und 48, die parallel zueinander und quer, insbesondere senkrecht, zu den Rahmenlängsabschnittslängsachsen 38 und 40 verlaufen. Die Rahmenquerabschnitte 42 und 44 sind vom Rahmen weg weisend außen im Wesentlichen konvex gekrümmt. Äußere Kanten der Rahmenquerabschnitte sind abgerundet, um eine Verletzungsgefahr für einen Operateur während eines Einsatzes der Vorrichtung 18 zu minimieren.

Die beiden Rahmenlängsabschnitte 34 und 36 und der Rahmenquerabschnitt 42 sind einstückig ausgebildet und bilden eine Grundeinheit des Rahmens 24. Freie Enden 50 der Rahmenlängsabschnitte 34 und 36 weisen vom Rahmenquerabschnitt 42 weg und sind mit in Richtung auf den Rahmenquerabschnitt 42 hin weisenden freien Enden 52 des Rahmenquerabschnitts 44 koppelbar. Der Rahmen 24 definiert so eine innere, freie, im Wesentlichen langgestreckt rechteckige Durchbrechung 54.

Die beiden Rahmenlängsabschnitte 34 und 36 umfassen zwei parallel zueinander verlaufende Schienenteile 56, die spiegelsymmetrisch zueinander ausgebildet sind und jeweils eine in Richtung auf das andere Schienenteil 56 hin weisend geöffnete Führungsnut 58 aufweisen. Die freien Enden 50 der Schienenteile 56 weisen jeweils ein Sackloch 60 auf, dessen Längsachse parallel zur Rahmenlängsabschnittslängsachse 38 verläuft. In die Sacklöcher 60 sind zylindrische Verbindungsbolzen 62 eingesetzt, die im Wesentlichen zur Hälfte über die Enden 50 vorstehen. Sie bilden Rahmenabschnittsverbindungsvorsprünge 64 einer insgesamt mit dem Bezugszeichen 66 bezeichneten Rahmenabschnittsverbindungseinrichtung 66. In den freien Enden 52 des Rahmenquerabschnitts 44 sind jeweils zwei Sacklöcher 68 ausgebildet, die in Richtung auf die Enden 50 hin weisend geöffnet sind und parallel zu den Rahmenlängsabschnittslängsachsen 38 und 40 verlaufende Längsachsen definieren.

Die Verbindungsbolzen 62 sind wahlweise im Sackloch 60 oder im Sackloch 68 befestigt, beispielsweise durch Kleben oder Schweißen. Die Verbindungsbolzen 62, die beispielsweise im Sackloch 60 festgelegt sind, bilden mit den Sacklöchern 68 zusammen erste und zweite zusammenwirkende Rahmenabschnittsverbindungsglieder 70 und 72 der Rahmenabschnittsverbindungseinrichtung 66, die in einer Rahmenverbindungsstellung, in welcher die den Rahmen 24 ausbildenden Teile, also beispielsweise die Rahmenteile 26 und 28, miteinander gekoppelt sind, kraft- und/oder formschlüssig miteinander in Eingriff stehen.

Die Sacklöcher 68 und die Verbindungsbolzen 62 können in ihren Innendurchmesser beziehungsweise Außendurchmesser so bemessen sein, dass die Rahmenteile 26 und 28 klemmend miteinander verbunden sind. Optional können in nicht dargestellter Weise auch Rastelemente oder Rastglieder an den Rahmenabschnittsverbindungsgliedern 70 beziehungsweise 72 angeordnet oder ausgebildet sein, um die Rahmenabschnittsverbindungseinrichtung 66 in Form einer Rast- und/oder Schnappverbindungseinrichtung auszubilden.

Der Rahmen 24 ist insgesamt modular ausgebildet und ermöglicht es, durch Verwendung von Rahmenabschnittsverlängerungen 74 vergrößert zu werden. Die Rahmenabschnittsverlängerungen 74 sind vorzugsweise identisch ausgebildet und umfassen jeweils zwei Schienenteile 76, die in analoger Weise wie die Schienenteile 56 aufeinander zu weisend geöffnete Führungsnuten 78 aufweisen.

Freie Enden 80 und 82 der sich parallel zu den Rahmenlängsabschnittslängsachsen 38 und 40 erstreckenden Rahmenabschnittsverlängerung 74 weisen Sacklöcher 84 und 86 auf, die analog zu den Sacklöchern 60 ausgebildet sind und parallel zu den Rahmenlängsabschnittslängsachsen 38 und 40 verlaufende Längsachsen definieren. In die Sacklöcher 86 sind wiederum mit den Verbindungsbolzen 62 identische Verbindungsbolzen 88 eingesetzt und klemmend gehalten.

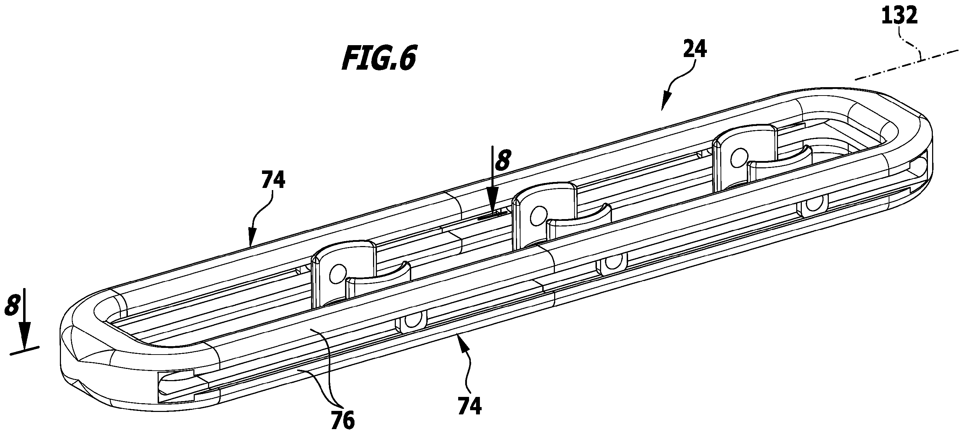

Die Ausbildung der Sacklöcher 84 und 86 sowie die Anordnung der Verbindungsbolzen 88 in den Sacklöchern 86 gestattet es, den Rahmenteil 28 mit freien Enden der Rahmenabschnittsverlängerungen 74 zu koppeln, von denen die Verbindungsbolzen 88 abstehen. Der Rahmenteil 28 mit den Rahmenabschnittsverlängerungen 74 kann nun mit dem Rahmenteil 26 gekoppelt werden, und zwar indem die Verbindungsbolzen 62 in die Sacklöcher 84 eingeschoben werden. Insgesamt kann so der Rahmen 24 bei Bedarf vergrößert werden.
Insbesondere ist es möglich, nicht nur zwei Rahmenabschnittsverlängerungen 74 vorzusehen, sondern noch weitere, die dann in analoger Weise miteinander und den Rahmenteilen 26 und 28 verbunden werden können. Ebenso ist es möglich, unterschiedlich lange Rahmenabschnittsverlängerungen 74 vorzusehen, um die Größe des Rahmens 24 praktisch beliebig anpassen zu können. Bei dem in den Figuren 6 bis 8 dargestellten Rahmen 24 entspricht eine Länge der Rahmenabschnittsverlängerung 74 in etwa einer Länge der Rahmenlängsabschnitte 34 und 36.
Die Vorrichtung 18, wie sie beispielhaft in den Figuren 1 bis 8 dargestellt ist, umfasst ferner drei Kopplungseinrichtungen 90, die jeweils eine Kopplungslängsachse 92 definieren. Sie sind ausgebildet, um die Instrumente 12 im Bereich von deren proximalen Enden 94 zu koppeln. Die Kopplungslängsachsen 92 der Kopplungseinrichtungen 90 sind parallel zueinander ausrichtbar.
Jede Kopplungseinrichtung 90 umfasst zwei Kopplungsglieder 96. Jedes Kopplungsglied 96 umfasst einen Führungsabschnitt 98, einen Halteabschnitt 100 und ein den Führungsabschnitt 98 und den Halteabschnitt 100 verbindendes Verbindungsglied 102.
Der Führungsabschnitt 98 ist im Wesentlichen in Form einer flachen quadratischen Platte 104 ausgebildet, welche eine Höhe 106 aufweist, die im Wesentlichen einem Abstand von aufeinander zu weisenden Nutflächen 108 der Führungsnuten 58 an den Schienenteilen 56 entspricht. Breiten 110 und 112 der Führungsnuten 58 an den Rahmenlängsabschnitten 34 und 36 sind jeweils etwas größer als eine Dicke 114 des Führungsabschnitts 98.

Der Halteabschnitt 100 ist in Form eines Ausschnitts einer hohlzylindrischen Wand ausgebildet und weist eine in Richtung auf den gegenüberliegenden Halteabschnitt 100 hin weisende Instrumentenanlagefläche 116 auf, die an eine äußere Kontur des medizinischen Instruments 12 im Bereich von dessen proximalem Ende 94 angepasst ist. Vorzugsweise ist die Instrumentenanlagefläche 116 in Form eines hohlzylindrischen Wandflächenabschnitts 118 ausgebildet. Der Führungsabschnitt 98, das Verbindungsglied 102 und der Halteabschnitt 100 sind unbeweglich miteinander verbunden. Der Halteabschnitt 100 ist so bezogen auf den Führungsabschnitt 98 ausgerichtet, dass die Kopplungslängsachsen 92, die insbesondere Mittelachsen der Wandflächenabschnitte 118 bilden, senkrecht zu den Rahmenlängsabschnittslängsachsen 38 und 40 sowie senkrecht zu den Rahmenquerabschnittslängsachsen 46 und 48 verlaufen.

Zum beweglichen Verbinden der Kopplungsglieder 96 mit dem Rahmen 24 dient eine Verbindungseinrichtung 120. Diese umfasst erste und zweite Verbindungselemente 122 und 124, wobei das erste Verbindungselement 122 am Rahmen 24 angeordnet beziehungsweise ausgebildet ist und das zweite Verbindungselement 124 am Kopplungsglied 96. Das erste Verbindungselement 122 ist in Form einer Verbindungsaufnahme 126 ausgebildet, die durch die beiden Führungsnuten 58 beziehungsweise 78 gebildet wird.

Das zweite Verbindungselement 124 ist in Form eines Verbindungsvorsprungs 128 ausgebildet, und zwar in Form des Führungsabschnitts 98. Der Führungsabschnitt 98 ist in der Montagestellung in der Verbindungsaufnahme 126 verschiebbar, jedoch nicht relativ zu dieser verdrehbar. Die Verbindungsaufnahme 126 bildet einen Teil der Schienenteile 56 beziehungsweise 76 und umfasst die Führungsnuten 58 beziehungsweise 78. Die Führungsschienen 130 werden gebildet durch die paarweisen Schienenteile 56 der Rahmenlängsabschnitte 34 und 36 beziehungsweise der Rahmenabschnittsverlängerungen 74. Insgesamt weist somit die Verbindungseinrichtung 120 zwei parallel zueinander verlaufende Verbindungsaufnahmen 126 auf.

Dadurch, dass die Führungsabschnitte 98 schmaler sind als die Führungsnuten 58 und 78 breit sind, können die Kopplungsglieder 96 relativ zueinander in einer Richtung quer zu einer von den ersten Verbindungselementen 122 definierten Längsrichtung 132, also parallel zu den Rahmenquerabschnittslängsachsen 46 und 48, bewegt werden. Eine Bewegung der Kopplungsglieder 96 aufeinander zu wird dadurch begrenzt, dass in Richtung auf die Halteabschnitte 100 weisende Anschlagflächen 134 der Führungsabschnitte 98 an von den Halteabschnitten 100 weg weisenden Seitenflächen 136 der Führungsnuten 58 beziehungsweise 78 anschlagen.

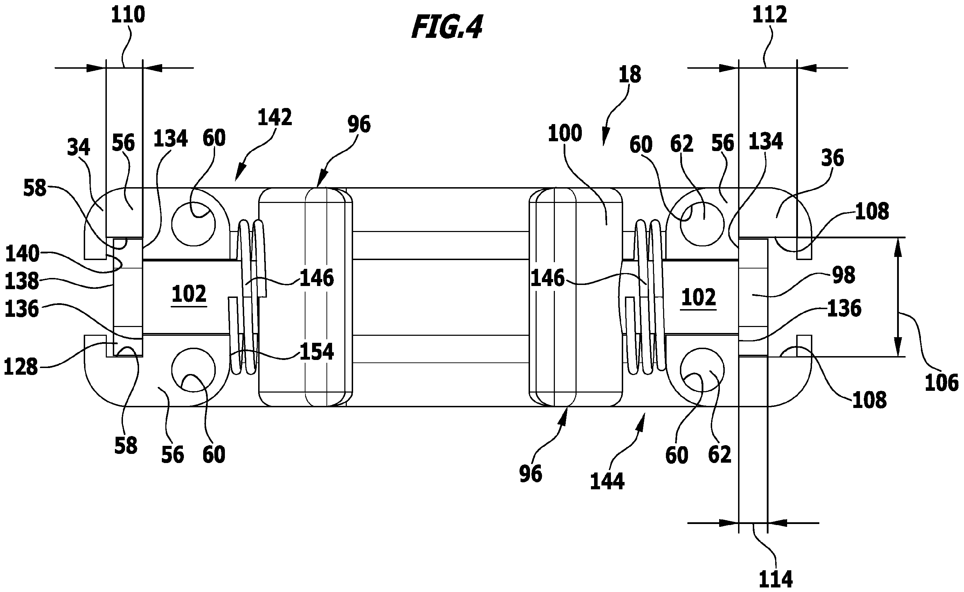

Eine Bewegung der Kopplungsglieder 96 voneinander weg wird dadurch begrenzt, dass eine vom Halteabschnitt 100 weg weisende Seitenfläche 138 des Führungsabschnitts 98 an in Richtung auf den Halteabschnitt 100 weisenden Seitenflächen 140 der Führungsnuten 58 beziehungsweise 78 anschlägt. Sind die beiden aufeinander zu weisenden Halteabschnitte 100 der beiden eine Kopplungseinrichtung 90 ausbildenden Kopplungsglieder 96 maximal aufeinander zu bewegt, so nehmen sie die Ausrichtstellung ein, die beispielsweise in der Figur 4 schematisch dargestellt ist. Werden sie aus der Ausrichtstellung ausgelenkt, um einen Abstand zwischen den Instrumentenanlageflächen 116 zu vergrößern, wird die Kopplungseinrichtung 90 von der Ausrichtstellung in die Montagestellung überführt. In dieser ist es auf einfache Weise möglich, den Rahmen 24 beispielsweise über die Enden 94 der Instrumente 12 zu führen, welche bereits mit den nicht dargestellten Befestigungseinrichtungen gekoppelt sind.

Die Kopplungseinrichtung 90 umfasst ferner eine Feststelleinrichtung 142 zum Sichern einer Position und/oder Ausrichtung der Kopplungsglieder 96 in der Ausrichtstellung. Sie ist beispielsweise bei dem in den Figuren 1 bis 8 schematisch dargestellten Ausführungsbeispiel einer Vorrichtung 18 in Form einer Klemmeinrichtung 144 ausgebildet zum klemmenden Festlegen der Kopplungsglieder 96 in der Ausrichtstellung am Rahmen 24.

Die Klemmeinrichtung 144 umfasst ein Klemmglied 146 zum klemmenden Festlegen der Kopplungsglieder 96 in der Ausrichtstellung am Rahmen 24. Jedem Kopplungsglied 96 ist ein Klemmglied 146 zugeordnet, welches in Form eines Federelements 148 ausgebildet ist. Dieses wird bei der Vorrichtung 18, die beispielhaft in den Figuren 1 bis 8 dargestellt ist, durch eine Schraubenfeder 150 gebildet, die als Druckglied 152 dient.

Das Klemmglied 146 stützt sich einerseits am Halteabschnitt 100 und andererseits an einer Seitenfläche 154 der Rahmenlängsabschnitte 34 und 36 beziehungsweise der Rahmenabschnittsverlängerungen 74 ab. Damit nimmt das Kopplungsglied 96 in einer Grundstellung die Ausrichtstellung ein. Das Kopplungsglied 96 kann jedoch entgegen der Wirkung des Klemmglieds 146 von der Ausrichtstellung in die Montagestellung überführt werden. Dabei wird das Druckglied 152 komprimiert. Alternativ könnte das das Verbindungsglied 102 umgebende Klemmglied 146 auch derart angeordnet sein, dass es sich das Verbindungsglied 102 umgebend einerseits an der Seitenfläche 136 und andererseits an der Anschlagfläche 134 abstützt.

In der Ausrichtstellung drückt das Klemmglied 146 den Führungsabschnitt 98 gegen die Seitenflächen 136, so dass es nur sehr schwer oder praktisch unmöglich ist, die Kopplungsglieder 96 in der Verbindungsaufnahme 126 zu verschieben. Werden die Kopplungsglieder 96 jedoch in die Montagestellung überführt, so dass die Führungsabschnitte 98 im Wesentlichen weder an den Seitenflächen 136 noch an den Seitenflächen 140 anliegen, dann können die Kopplungsglieder 96 in der Verbindungsaufnahme 126 parallel zur Längsrichtung 132 verschoben werden. Dies ermöglicht es, die Kopplungsglieder 96 und damit die Kopplungseinrichtungen 90 am Rahmen 24 so zu positionieren, dass die Instrumente 12 in gewünschter Weise, das heißt parallel zueinander ausgerichtet, am Rahmen 24 in den Kopplungseinrichtungen 90 festgelegt werden können.

Die Zahl der am Rahmen 24 anordenbaren Kopplungseinrichtungen 90 kann auf einfache Weise geändert werden, indem entweder Kopplungsglieder 96 entfernt oder hinzugefügt werden. Dies kann am einfachsten erfolgen, wenn beispielsweise der Rahmenteil 28 vom Rahmenteil 26 getrennt ist und die Enden 50 freiliegen, so dass weitere Kopplungsglieder 96 mit ihren Führungsabschnitten 98 in die Verbindungsaufnahmen 126 hineingeschoben werden können.

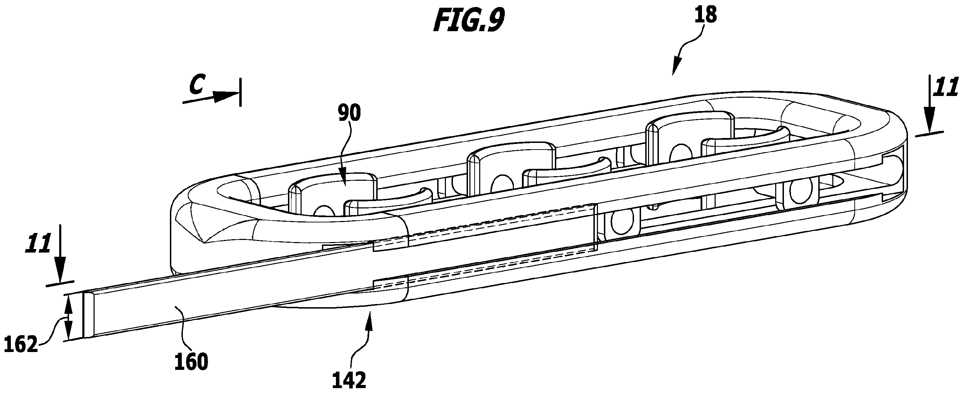

In den Figuren 9 bis 11 ist ein weiteres Ausführungsbeispiel einer medizinischen Vorrichtung 18 schematisch dargestellt. Sie stimmt in ihrem Aufbau mit dem in den Figuren 1 bis 8 schematisch dargestellten Ausführungsbeispiel einer Vorrichtung 18 praktisch überein. Sie unterscheidet sich lediglich dadurch, dass sie keine Klemmeinrichtung 144 aufweist, sondern eine Feststelleinrichtung 142 in Form einer Blockiereinrichtung 156.

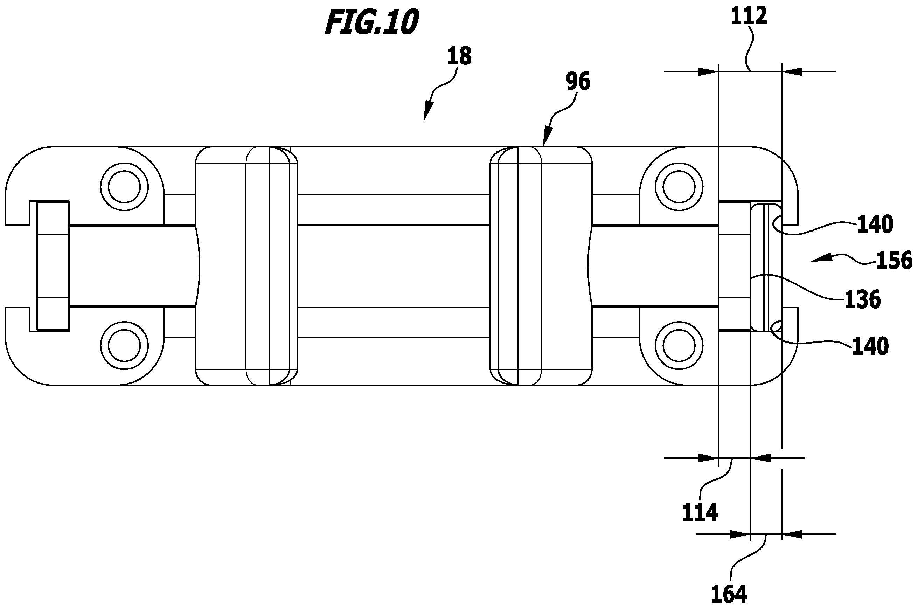

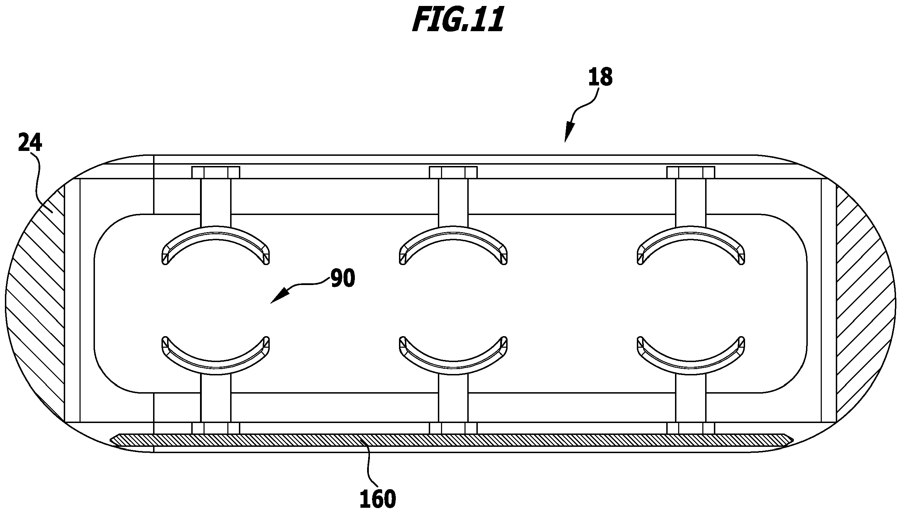

Die Blockiereinrichtung 156 umfasst ein Blockierelement 160, welches in Form eines langgestreckten quaderförmigen Streifens 160 aus einem Metall oder einem im Wesentlichen inelastischen Kunststoff gebildet ist. Eine Breite 162 des Streifens 160 entspricht der Höhe 106. Eine Dicke 164 entspricht zusammen mit der Dicke 114 des Führungsabschnitts 98 einer Breite 112 der Führungsnut 58 beziehungsweise 78. Dies ermöglicht es, das Blockierelement 160 in die Verbindungsaufnahme 126 einzuschieben zwischen die Seitenfläche 136 des Führungsabschnitts 98 und die Seitenflächen 140 der Führungsnuten 58 beziehungsweise 78.
Ist der Streifen 160 in die Verbindungsaufnahme 126 in der beschriebenen Weise eingeschoben, wie dies schematisch in den Figuren 10 und 11 dargestellt ist, kann das mit dem Blockierelement 160 zusammenwirkende Kopplungsglied 96 nicht mehr relativ zum anderen Kopplungsglied 96 der Kopplungseinrichtung 90 bewegt werden. Damit blockiert die Blockiereinrichtung 156 die Kopplungseinrichtung 90 in der Ausrichtstellung.

Um die Kopplungseinrichtung 90 von der Ausrichtstellung in die Montagestellung zu überführen, wird das Blockierelement 160 aus der Verbindungsaufnahme 126 herausgezogen, so dass die Kopplungsglieder 96 in der Führungsaufnahme 100 wieder parallel zur Längsrichtung 132 verschoben werden können. Optional können die Kopplungsglieder 96, die nicht direkt mit dem Blockierelement 156 in Kontakt stehen, wie bei dem in den Figuren 1 bis 8 dargestellten Ausführungsbeispiel einer Vorrichtung 18, auch ein Klemmglied 146 umfassen.

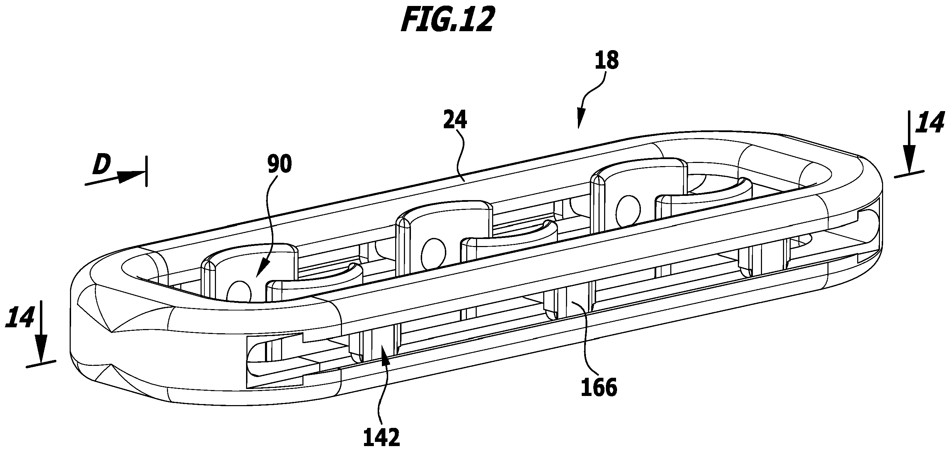

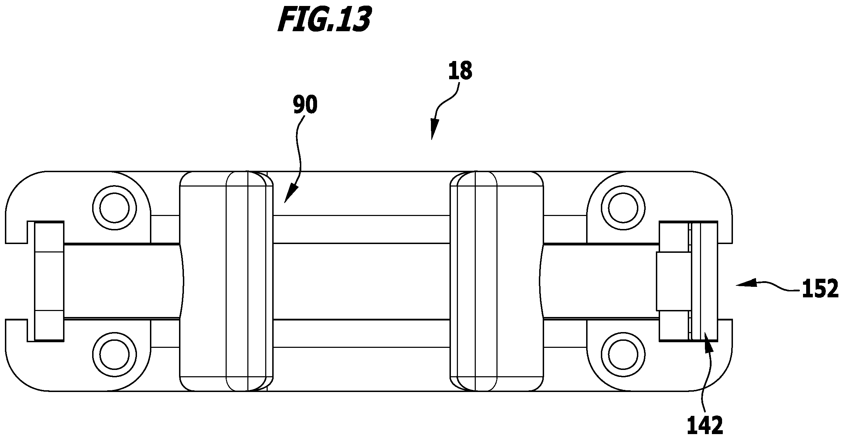

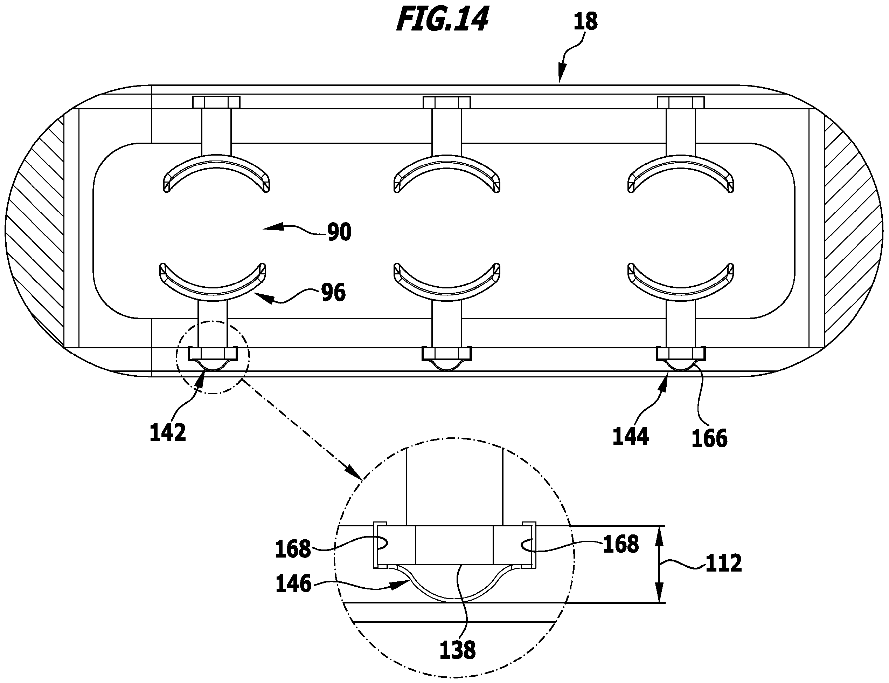

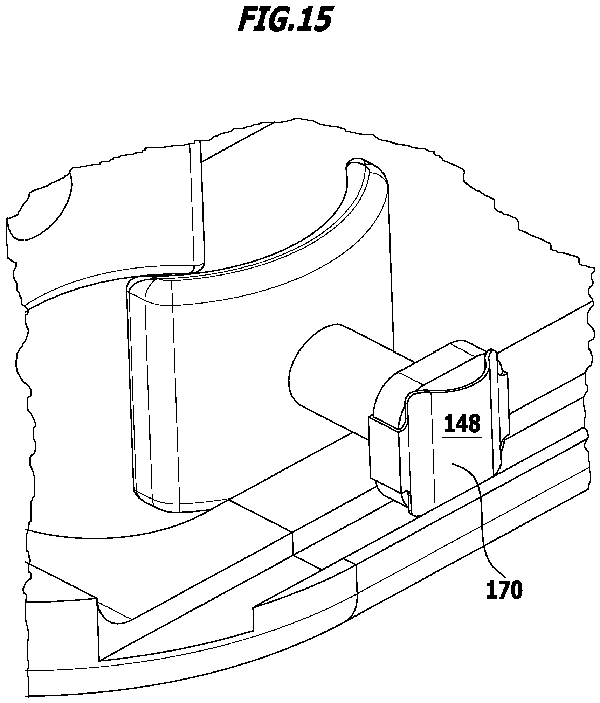

In den Figuren 12 bis 15 ist schematisch ein weiteres Ausführungsbeispiel einer medizinischen Vorrichtung 18 schematisch dargestellt. Der grundsätzliche Aufbau der Vorrichtung 18 stimmt mit dem Aufbau der in den Figuren 1 bis 8 schematisch dargestellten Vorrichtung 18 überein. Der Unterschied besteht allein in der Ausgestaltung der Feststelleinrichtung 142, die zwar ebenfalls in Form einer Klemmeinrichtung 144 ausgebildet ist, sich aber durch die in den Führungsnuten 58 beziehungsweise 78 mit der Breite 112 geführten Kopplungsgliedern 98 zusammenwirkenden Klemmgliedern 146 unterscheidet.
Die Klemmglieder 146 sind zwar ebenfalls als Federelemente 148 ausgebildet, jedoch in Form von Blattfedern 166, die auf die Führungsabschnitte 98 aufgeklemmt sind. Jede Blattfeder 166 ist bezüglich einer parallel zu den Rahmenquerschnittslängsachsen 46 und 48 verlaufenden Spiegelebene spiegelsymmetrisch ausgebildet. Sie weist zwei freie Enden auf, die zur Ausbildung von Ausnehmungen 168 umgebogen sind, so dass die Ausnehmungen 168 aufeinander zu weisen und voneinander weg weisende Seitenflächen des Führungsabschnitts 98, die parallel zur Längsrichtung 132 weisen, umgreifen.
Von der Seitenfläche 138 weg weisend ist die Blattfeder 166 weg gewölbt und liegt mit einer konvex vom Führungsabschnitt 98 weg weisenden Außenfläche 170 an den Seitenflächen 140 der Führungsnuten 58 beziehungsweise 78 an. Die als Druckglied 152 ausgebildete Blattfeder 166 übt eine von der Seitenfläche 140 weg weisende Kraft auf den Führungsabschnitt 98 auf und hält so das Kopplungsglied 96 in Richtung auf das gegenüberliegende Kopplungsglied 96 vorgespannt. Mit anderen Worten hält die Klemmeinrichtung 144 die Kopplungseinrichtung 90 in der Ausrichtstellung.

Zum Überführen der Kopplungseinrichtung 90 von der Ausrichtstellung in die Montagestellung kann das Kopplungsglied 90 entgegen der Wirkung der Blattfeder 166 mit der Seitenfläche 138 in Richtung auf die Seitenfläche 140 verschoben werden. Dies ermöglicht dann auch eine Verschiebung des Führungsabschnitts 98 und damit des Klemmglieds 96 parallel zur Längsrichtung 132 in der Verbindungsaufnahme 126. Die Kopplungsglieder 96 können so in gewünschter Weise zur Aufnahme insbesondere proximaler Enden der Instrumente 12 am Rahmen 24 verschoben werden.

Die in den Figuren 12 bis 15 dargestellten und beschriebenen Kopplungsglieder 96 können grundsätzlich auch gegen die Kopplungsglieder 96 des in Figuren 1 bis 8 schematisch dargestellten Ausführungsbeispiels einer medizinischen Vorrichtung 18 ausgetauscht werden.

Insbesondere der Rahmen 24 und auch die anderen Teile der oben beschriebenen Ausführungsbeispiele medizinischer Vorrichtungen 18 sind vorzugsweise aus sterilisierbaren, insbesondere heißdampfsterilisierbaren Materialien ausgebildet. Aus Stabilitätsgründen ist es günstig, wenn es sich bei diesen Materialen um Metall oder Metalllegierungen handelt. Denkbar ist es aber auch, Kunststoffe zu nutzen, die entsprechende Festigkeiten für den Verwendungszweck der Vorrichtungen 18 aufweisen.

## Patentansprüche

1. Medizinische Vorrichtung (18) zum parallelen Ausrichten von mindestens zwei eine Instrumentenlängsachse (20) definierenden medizinischen Instrumenten (12) zum Halten und Handhaben eines chirurgischen Befestigungselements, welches Befestigungselement einen Befestigungsteil und einen relativ zum Befestigungsteil in einer Montagestellung beweglich gelagerten Halteteil für ein Verbindungselement umfasst, welche medizinischen Instrumente (12) ein distales, mit dem Befestigungselement koppelbares Ende (16) aufweisen, welche Vorrichtung (18) mindestens zwei, jeweils eine Kopplungslängsachse (92) definierende Kopplungseinrichtungen (90) zum temporären Koppeln mit proximalen Enden (94) der mindestens zwei medizinischen Instrumente (12) umfasst, wobei die Vorrichtung (18) einen Rahmen (24) umfasst, wobei der Rahmen (24) einen ersten und einen zweiten Rahmenabschnitt (30, 32) umfasst, welche parallel zueinander verlaufen, wobei die Kopplungslängsachsen (92) der mindestens zwei Kopplungseinrichtungen (90) parallel zueinander ausrichtbar sind, wobei die mindestens zwei Kopplungseinrichtungen (90) in einer Montagestellung relativ zueinander und am Rahmen (24) bewegbar gehalten sind und wobei sie in einer Ausrichtstellung relativ zueinander und am Rahmen (24) unbewegbar gehalten sind, **dadurch gekennzeichnet, dass** der Rahmen (24) modular ausgebildet ist und mindestens eine Rahmenabschnittsverlängerung (74) zum Verlängern der ersten und zweiten Rahmenabschnitte (30, 32) umfasst und dass die mindestens eine Rahmenabschnittsverlängerung (74) mit dem ersten und/oder zweiten Rahmenabschnitt (30, 32) lösbar verbindbar ist.

2. Vorrichtung nach Anspruch 1, **dadurch gekennzeichnet, dass** jede der Kopplungseinrichtungen (90) mindestens zwei Kopplungsglieder (96) umfasst, die in der Montagestellung relativ zueinander bewegbar und am Rahmen (24) bewegbar gehalten sind und die in der Ausrichtstellung relativ zueinander unbewegbar und am Rahmen (24) unbewegbar gehalten sind.

3. Vorrichtung nach einem der voranstehenden Ansprüche, **gekennzeichnet durch** eine Rahmenabschnittsverbindungseinrichtung (66) zum lösbaren Verbinden von den Rahmen (24) ausbildenden Teilen desselben.

4. Vorrichtung nach Anspruch 2 oder 3, **gekennzeichnet durch** eine Verbindungseinrichtung (120) zum beweglichen Verbinden der Kopplungsglieder (96) mit dem Rahmen (24).

5. Vorrichtung nach Anspruch 4, **dadurch gekennzeichnet, dass** die Verbindungseinrichtung (120) mindestens ein erstes Verbindungselement (122) und mindestens ein zweites, mit dem mindestens einen ersten Verbindungselement (122) zusammenwirkendes Verbindungselement (124) umfasst, dass der Rahmen (24) das mindestens eine erste Verbindungselement (122) umfasst und dass das mindestens eine zweite Verbindungselement (124) am Kopplungsglied (96) angeordnet oder ausgebildet ist.

6. Vorrichtung nach Anspruch 5, **dadurch gekennzeichnet, dass** die beiden eine Kopplungseinrichtung (90) definierenden Kopplungsglieder (96) relativ zueinander in einer Richtung quer zu einer von den mindestens einen ersten Verbindungselementen (122) definierten Längsrichtung (132) beim Übergang von der Ausrichtstellung in die Montagestellung voneinander weg und beim Übergang von der Montagestellung in die Ausrichtstellung aufeinander zu bewegbar sind.

7. Vorrichtung nach einem der Ansprüche 2 bis 6, **dadurch gekennzeichnet, dass** die Kopplungseinrichtung (90) eine Feststelleinrichtung (142) umfasst zum Sichern einer Position und/oder Ausrichtung der Kopplungsglieder (96) in der Ausrichtstellung.

8. Vorrichtung nach Anspruch 7, **dadurch gekennzeichnet, dass** die Feststelleinrichtung (142) eine Klemm- oder Blockiereinrichtung (144; 156) umfasst zum klemmenden Festlegen der Kopplungsglieder (96) in der Ausrichtstellung am Rahmen (24) oder zum Blockieren einer Bewegung derselben relativ zueinander.

9. Vorrichtung nach Anspruch 8, **dadurch gekennzeichnet, dass** die Klemmeinrichtung (144) mindestens ein Klemmglied (146) umfasst zum klemmenden Festlegen der Kopplungsglieder (96) in der Ausrichtstellung am Rahmen (24), wobei insbesondere das mindestens eine Klemmglied (146) in Form eines Federelements (148) ausgebildet ist.

10. Vorrichtung nach Anspruch 9, **dadurch gekennzeichnet, dass** mindestens ein Kopplungsglied (96) jeder Kopplungseinrichtung (90) entgegen der Wirkung des Klemmglieds (146) von der Ausrichtstellung in die Montagestellung überführbar ist.

11. Vorrichtung nach Anspruch 9 oder 10, **dadurch gekennzeichnet, dass** nur eines der beiden Kopplungsglieder (96) einer Kopplungseinrichtung (90) ein Klemmglied (146) umfasst.

12. Vorrichtung nach einem der Ansprüche 2 bis 11, **dadurch gekennzeichnet, dass** mindestens eines der Kopplungsglieder (96) einen Führungsabschnitt (98), einen Halteabschnitt (100) und ein den Führungsabschnitt (98) und den Haltabschnitt (100) verbindendes Verbindungsglied (102) aufweist.

13. Vorrichtung nach einem der Ansprüche 8 bis 12, **dadurch gekennzeichnet, dass** das mindestens eine erste Verbindungselement (122) in Form einer Verbindungsaufnahme (126) ausgebildet ist und dass das mindestens eine zweite Verbindungselement (124) in Form eines Verbindungsvorsprungs (128) ausgebildet ist, dass die Blockiereinrichtung (156) ein Blockierelement (160) umfasst, welches in der Montagestellung in die Verbindungsaufnahme (126) einschiebbar ist und in der Ausrichtstellung zwischen einer vom Halteabschnitt (100) weg weisenden Seitenfläche (138) des Führungsabschnitts (98) und einer inneren Führungsfläche (140) der Verbindungsaufnahme (126) angeordnet ist.

14. Medizinisches Instrumentarium (22) zum Implantieren eines Wirbelsäulenstabilisierungssystems (10), welches Instrumentarium (22) mindestens zwei medizinische Instrumente (12) zum Halten und Handhaben eines chirurgischen Befestigungselements umfasst, welches Befestigungselement einen Befestigungsteil und einen relativ zum Befestigungsteil in einer Montagestellung beweglich gelagerten Halteteil für ein Verbindungselement umfasst, welche medizinischen Instrumente (12) ein proximales und ein distales, mit dem Befestigungselement koppelbares Ende (16) aufweisen, **gekennzeichnet durch** eine Vorrichtung (18) nach einem der voranstehenden Ansprüche.

15. Wirbelsäulenstabilisierungssystem (10) umfassend mindestens zwei chirurgische Befestigungselemente und mindestens ein Verbindungselement, wobei mindestens eines der mindestens zwei chirurgischen Befestigungselemente einen Befestigungsteil, einen Halteteil mit einer Verbindungselementaufnahme und ein am Halteteil festlegbares Fixierelement zum Festlegen des Verbindungselements in der Verbindungselementaufnahme umfasst, **gekennzeichnet durch** eine Vorrichtung (18) nach einem der Ansprüche 1 bis 13 oder durch ein medizinisches Instrumentarium (22) nach Anspruch 14.

## Claims

1. Medical apparatus (18) for parallel alignment of at least two medical instruments (12), defining an instrument longitudinal axis (20), for holding and manipulating a surgical fastening element, which fastening element comprises a fastening part and a holding part for a connection element, the holding part being mounted movably relative to the fastening part in a mounting position, which medical instruments (12) have a distal end (16) couplable to the fastening element, which apparatus (18) comprises at least two coupling devices (90), each defining a coupling longitudinal axis (92), for temporary coupling with proximal ends (94) of the at least two medical instruments (12), wherein the apparatus (18) comprises a frame (24), wherein the frame (24) comprises a first and a second frame portion (30, 32), which extend parallel to one another, wherein the coupling longitudinal axes (92) of the at least two coupling devices (90) are alignable parallel to one another, wherein in a mounting position the at least two coupling devices (90) are held in such a way as to be movable relative to one another and on the frame (24) and wherein in an alignment position they are held immovably relative to one another and on the frame (24), **characterised in that** the frame (24) is of modular construction and comprises at least one frame portion extension (74) for extending the first and second frame portions (30, 32) and **in that** the at least one frame portion extension (74) is detachably connectable to the first and/or second frame portion (30, 32).

2. Apparatus according to claim 1, **characterised in that** each of the coupling devices (90) comprises at least two coupling members (96), which in the mounting position are held so as to be movable relative to one another and on the frame (24) and which in the alignment position are held immovably relative to one another and on the frame (24).

3. Apparatus according to any one of the preceding claims, **characterized by** a frame portion connection device (66) for detachable connection of parts forming the frame (24).

4. Apparatus according to claim 2 or claim 3, **characterized by** a connection device (120) for movable connection of the coupling members (96) with the frame (24).

5. Apparatus according to claim 4, **characterised in that** the connection device (120) comprises at least one first connection element (122) and at least one second connection element (124) interacting with the at least one first connection element (122), **in that** the frame (24) comprises the at least one first connection element (122) and **in that** the at least one second connection element (124) is arranged or formed on the coupling member (96).

6. Apparatus according to claim 5, **characterised in that** the two coupling members (96) defining a coupling device (90) are movable relative to one another in a direction transverse to a longitudinal direction (132) defined by the at least one first connection elements (122) away from one another on changeover from the alignment position to the mounting position and towards one another on changeover from the mounting position to the alignment position.

7. Apparatus according to any one of claims 2 to 6, **characterised in that** the coupling device (90) comprises a locking device (142) for securing a position and/or alignment of the coupling members (96) in the alignment position.

8. Apparatus according to claim 7, **characterised in that** the locking device (142) comprises a clamping or blocking device (144; 156) for clamping securing of the coupling members (96) on the frame (24) in the alignment position or for blocking movement thereof relative to one another.

9. Apparatus according to claim 8, **characterised in that** the clamping device (144) comprises at least one clamping member (146) for clamping securing of the coupling members (96) on the frame (24) in the alignment position, wherein in particular the at least one clamping member (146) takes the form of a spring element (148).

10. Apparatus according to claim 9, **characterised in that** at least one coupling member (96) of each coupling device (90) is transferable from the alignment position into the mounting position against the action of the clamping member (146).

11. Apparatus according to claim 9 or claim 10, **characterised in that** just one of the two coupling members (96) of a coupling device (90) comprises a clamping member (146).

12. Apparatus according to any one of claims 2 to 11, **characterised in that** at least one of the coupling members (96) has a guide portion (98), a holding portion (100) and a connecting member (102) connecting the guide portion (98) and the holding portion (100).

13. Apparatus according to any one of claims 8 to 12, **characterised in that** the at least one first connection element (122) takes the form of a connection receptacle (126) and **in that** the at least one second connection element (124) takes the form of a connecting projection (128), that the blocking device (156) comprises a blocking element (160) which is insertable into the connecting receptacle (126) in the mounting position and in the alignment position is arranged between a side face (138) of the guide portion (98) facing away from the holding portion (100) and an inner guide face (140) of the connecting recess (126).

14. Set (22) of medical instruments for implanting a spine stabilisation system (10), which set (22) of medical instruments comprises at least two medical instruments (12) for holding and manipulating a surgical fastening element, which fastening element comprises a fastening part and a holding part for a connection element, the holding part being mounted movably relative to the fastening part in a mounting position, which medical instruments (12) have a proximal end and a distal end (16) couplable to the fastening element, **characterised by** an apparatus (18) according to any one of the preceding claims.

15. Spine stabilisation system (10) comprising at least two surgical fastening elements and at least one connection element, at least one of the at least two surgical fastening elements comprising a fastening part, a holding part with a connection element receptacle and a fixing element securable to the holding part for securing the connection element in the connection element receptacle, **characterised by** an apparatus (18) according to any one of claims 1 to 13 or by a set of medical instruments (22) according to claim 14.

## Revendications

1. Dispositif médical (18) pour assurer l'orientation parallèle d'au moins deux instruments médicaux (12) définissant un axe longitudinal d'instrument (20) pour le maintien et la manipulation d'un élément de fixation chirurgical, ledit élément de fixation comprenant une partie de fixation et une partie de maintien pour un élément de liaison, montée mobile par rapport à la partie de fixation dans une position de montage, lesdits instruments médicaux (12) présentant une extrémité distale (16) pouvant être couplée à l'élément de fixation, ledit dispositif (18) comprenant au moins deux dispositifs de couplage (90) définissant chacun respectivement un axe longitudinal de couplage (92) et destinés au couplage temporaire avec des extrémités proximales (94) desdits au moins deux instruments médicaux (12), dispositif (18)
dans lequel est prévu un cadre (24),
dans lequel le cadre (24) comprend un premier et un deuxième tronçon de cadre (30, 32), qui s'étendent parallèlement l'un à l'autre,
dans lequel les axes longitudinaux de couplage (92) desdits au moins deux dispositifs de couplage (90) peuvent être orientés de manière à être mutuellement parallèles,
dans lequel lesdits au moins deux dispositifs de couplage (90) sont, dans une position de montage, maintenus mobiles les uns par rapport aux autres et sur le cadre (24), et sont, dans une position d'orientation, maintenus non mobiles les uns par rapport aux autres et sur le cadre (24),
**caractérisé en ce que** le cadre (24) est de configuration modulaire, et comprend au moins une rallonge de tronçon de cadre (74) pour rallonger les premier et deuxième tronçon de cadre (30, 32), et **en ce que** ladite au moins une rallonge de tronçon de cadre (74) peut être reliée de manière amovible au premier et/ou au deuxième tronçon de cadre (30, 32).

2. Dispositif selon la revendication 1, **caractérisé en ce que** chacun des dispositifs de couplage (90) comprend au moins deux organes de couplage (96), qui, dans la position de montage sont mobiles les uns par rapport aux autres et maintenus de manière mobile sur le cadre (24), et qui, dans la position d'orientation, sont non mobiles les uns par rapport aux autres et maintenus de manière non mobile sur le cadre (24).

3. Dispositif selon l'une des revendications précédentes, **caractérisé par** un dispositif de liaison de tronçons de cadre (66) pour assurer la liaison démontable de parties formant le cadre (24).

4. Dispositif selon la revendication 2 ou la revendication 3, **caractérisé par** un dispositif de liaison (120) pour assurer la liaison mobile des organes de couplage (96) avec le cadre (24).

5. Dispositif selon la revendication 4, **caractérisé en ce que** le dispositif de liaison (120) comprend au moins un premier élément de liaison (122) et au moins un deuxième élément de liaison (124), qui interagit avec ledit au moins un premier élément de liaison (122), **en ce que** le cadre (24) comporte ledit au moins un premier élément de liaison (122), et **en ce que** le deuxième élément de liaison (124) est agencé ou formé sur l'organe de couplage (96).

6. Dispositif selon la revendication 5, **caractérisé en ce que** les deux organes de couplage (96) définissant un dispositif de couplage (90) sont mobiles mutuellement l'un par rapport à l'autre dans une direction transversale à une direction longitudinale (132) définie par lesdits au moins un premiers éléments de liaison (122), de manière à s'éloigner l'un de l'autre lors du passage de la position d'orientation à la position de montage, et de manière à se rapprocher l'un de l'autre lors du passage de la position de montage à la position d'orientation.

7. Dispositif selon l'une des revendications 2 à 6, **caractérisé en ce que** le dispositif de couplage (90) comprend un dispositif d'immobilisation (142) pour sécuriser une position et/ou orientation des organes de couplage (96) dans la position d'orientation.

8. Dispositif selon la revendication 7, **caractérisé en ce que** le dispositif d'immobilisation (142) comprend un dispositif de serrage ou de blocage (144; 156) pour l'immobilisation par serrage des organes de couplage (96) dans la position d'orientation sur le cadre (24), ou bien pour le blocage d'un mouvement réciproque de ceux-ci.

9. Dispositif selon la revendication 8, **caractérisé en ce que** le dispositif de serrage (144) comprend au moins un organe de serrage (146) pour l'immobilisation par serrage des organes de couplage (96) dans la position d'orientation sur le cadre (24), ledit au moins un organe de serrage (146) étant notamment réalisé sous la forme d'un élément de ressort (148).

10. Dispositif selon la revendication 9, **caractérisé en ce qu'**au moins un organe de couplage (96) de chaque dispositif de couplage (90) peut être transféré de la position d'orientation à la position de montage, à l'encontre de l'action de l'organe de serrage (146).

11. Dispositif selon la revendication 9 ou la revendication 10, **caractérisé en ce que** seul l'un des deux organes de couplage (96) d'un dispositif de couplage (90) comprend un organe de serrage (146).

12. Dispositif selon l'une des revendications 2 à 11, **caractérisé en ce que** l'un au moins des organes de couplage (96) présente un tronçon de guidage (98), un tronçon de maintien (100) et un organe de liaison (102) qui relie le tronçon de guidage (98) et le tronçon de maintien (100).

13. Dispositif selon l'une des revendications 8 à 12, **caractérisé en ce que** ledit au moins un premier élément de liaison (122) est réalisé sous la forme d'un logement de liaison (126), et **en ce que** ledit au moins un deuxième élément de liaison (124) est réalisé sous la forme d'une protubérance de liaison (128), et **en ce que** le dispositif de blocage (156) comprend un élément de blocage (160), qui, dans la position de montage, peut être glissé dans le logement de liaison (126), et, dans la position d'orientation, est agencé entre une surface latérale (138) du tronçon de guidage (98), qui est dirigée à l'opposé d'où se trouve le tronçon de maintien (100), et une surface de guidage intérieure (140) du logement de liaison (126).

14. Panoplie d'instruments médicaux (22) pour implanter un système de stabilisation de colonne vertébrale (10), ladite panoplie d'instruments (22) comprenant au moins deux instruments médicaux (12) pour le maintien et la manipulation d'un élément de fixation chirurgical, ledit élément de fixation comprenant une partie de fixation et une partie de maintien pour un élément de liaison, montée mobile par rapport à la partie de fixation dans une position de montage, lesdits instruments médicaux (12) présentant une extrémité proximale et une extrémité distale (16) pouvant être couplée à l'élément de fixation, **caractérisée par** un dispositif (18) selon l'une des revendications précédentes.

15. Système de stabilisation de colonne vertébrale (10) comprenant au moins deux éléments de fixation chirurgicaux et au moins un élément de liaison, au moins l'un desdits au moins deux éléments de fixation chirurgicaux comportant une partie de fixation, une partie de maintien avec un logement d'élément de liaison et un élément de fixage pouvant être immobilisé sur la partie de maintien en vue d'immobiliser l'élément de liaison dans le logement d'élément de liaison, **caractérisé par** un dispositif (18) selon l'une des revendications 1 à 13 ou par une panoplie d'instruments médicaux (22) selon la revendication 14.
